# EUROPÄISCHE PATENTSCHRIFT

(11) **EP 2 105 098 B1**
(45) Veröffentlichungstag und Bekanntmachung des Hinweises auf die Patenterteilung: **27.10.2010**
(21) Anmeldenummer: 08005803.5
(22) Anmeldetag: 27.03.2008
(51) Int. Cl.: A61B 17/22, A61B 6/00

(54) **Druckwellentherapievorrichtung mit integrierter Röntgenanlage**
Pressure wave therapy device with integrated x-ray device
Dispositif de thérapie par ondes de pression doté d'une installation de radiologie intégrée

(43) Veröffentlichungstag der Anmeldung: 30.09.2009
(73) Patentinhaber: STORZ MEDICAL AG, 8274 Tägerwilen (CH)
(72) Erfinder: Wess Othmar, Dr, 8574 Lengwil Oberhofen (CH)
(74) Vertreter: Szynka, Dirk

(56) Entgegenhaltungen:
- EP-A- 0 369 177
- EP-A2- 0 288 698
- WO-A-00/15121
- WO-A-2004/006786
- DE-A1- 4 400 997
- DE-A1- 10 236 177
- DE-A1-102005 040 173

## Beschreibung

Die vorliegende Erfindung bezieht sich auf eine Druckwellentherapievorrichtung mit integrierter Röntgenanlage.

Druckwellen, d. h. mechanische und gelegentlich auch als "akustisch" bezeichnete Wellen werden in verschiedener Weise zur therapeutischen Behandlung eingesetzt. Besonders wichtig und historisch betrachtet auch Ausgangspunkt der Entwicklung ist die Stoßwellenlithotripsie, also die Zerkleinerung von Körperkonkrementen, insbesondere Steinen, mit fokussierten Druckwellen großer Amplituden und mit steilen Anstiegsflanken. Hier werden einzelne Pulse auf das Konkrement gerichtet, deren erste, einer Kompression entsprechende "Halbwelle" hinsichtlich Flankensteilheit und Amplitude sowie therapeutischer Wirkung dominiert, wohingegen bereits die nächste nachfolgende Halbwelle, die einer Expansion entspricht, deutlich weniger ausgeprägt ist. Solche Pulse werden regelmäßig wiederholt angewendet.

Vergleichbare Verfahren mit Stoßwellen sind auch für andere Indikationen bekannt, etwa zur Beaufschlagung von schlecht heilenden Knochenbrüchen.

Daneben betrifft die Erfindung aber auch Druckwellentherapien mit eigentlichen, also fortgesetzt oszillierenden Wellen. Diese können in fokussierter Weise zur Erwärmung von Körpergewebe eingesetzt werden, etwa zur sog. thermischen Ablation von Tumoren.

Obwohl auch Therapien mit nicht fokussierten Druckwellen bekannt sind, richtet sich die vorliegende Erfindung auf Anwendungen mit fokussierten Wellen (wobei Pulse inbegriffen sein sollen, vgl. oben). Wenngleich die Abgrenzung zwischen fokussierten und nicht fokussierten Wellen Schwierigkeiten bereiten kann, soll es im Folgenden jedenfalls nur um solche Therapien gehen, bei denen die Druckwellen bewusst auf eine mehr oder weniger ausgedehnte Körperregion konzentriert werden, um erhöhte Intensitäten, Drücke oder Flankensteilheiten zu erzielen.

Da bei diesen fokussierenden Therapien die Lokalisierung auf die zu behandelnde Körperregion naturgemäß wesentlich ist, spielt die Einstellung der entsprechenden Vorrichtungen zur richtigen Anordnung des Fokusbereichs im Körper eine entscheidende Rolle. Dies betrifft natürlich zum einen die anfängliche Ortung der und Einstellung auf die zu behandelnde Region, etwa einen Stein. Wenn hier zu große Toleranzen auftreten, wird gesundes Gewebe beschädigt oder unnötig viel gesundes Gewebe in Mitleidenschaft gezogen und andererseits der Therapieerfolg in der eigentlich zu behandelnden Region eingeschränkt oder gefährdet. Man spricht hier auch von der "Navigation".

Erschwerend kommt hinzu, dass die Navigation kein statischer Vorgang sein muss, also Änderungen während der Behandlung auftreten können. Eine wesentliche Ursache sind Bewegungen des Patienten oder Verschiebungen von Organen, insbesondere infolge seiner Atmung.

Zur Navigation können bildgebende Verfahren herangezogen werden, die die zu behandelnde Region von der Umgebung unterscheidbar machen und Navigationsinformationen dazu, also letztlich räumliche Koordinaten, für die Druckwellenvorrichtung zur Verfügung stellen können. Bekannt sind insbesondere die Röntgenortung und auch die Röntgenüberwachung während der Stoßwellenlithotripsie. Wenn beispielsweise der Patient auf einer Patientenliege positioniert worden ist, muss also zunächst genau ermittelt werden, wo die zu behandelnde Region liegt. Hierzu wird konventioneller Weise zunächst eine vertikale Röntgendurchleuchtung durchgeführt. In aller Regel wird die zu behandelnde Region, beispielsweise der Stein, in dem Röntgenbild erscheinen, aber nicht zentriert sein. Man kann dann eine horizontale Verschiebung vornehmen (entweder der Liege oder des Ständers mit der Röntgenanlage) bis der Stein zentriert ist.

Zur dreidimensionalen Ortung einer zu behandelnden Region sind grundsätzlich zwei verschiedene Röntgenaufnahmen aus verschiedenen Richtungen nötig, etwa in einem Winkel von 30° zueinander. Also wird eine zweite Röntgendurchleuchtung in einer zweiten Richtung vorgenommen, um die richtige Höhe etwa des Steins einstellen zu können, ihn also auf das sog. Isozentrum zu justieren. Hierzu kann beispielsweise die Patientenliege in der Höhe verstellt werden. Erst wenn der Stein dreidimensional zentriert ist und damit im Druckwellenfokus liegt, kann er desintegriert werden.

Grundsätzlich kann aber eine Röntgenaufnahrne in nur einer Richtung mit anderen bildgebenden Verfahren oder anderen nicht bildgebenden Ortungsmöglichkeiten kombiniert werden. Es kann auch Fälle geben, in denen aus anatomischen Gründen eine zweidimensionale Ortung mit einer Röntgenabbildungsrichtung ausreicht.

Jedenfalls ist häufig eine kombinierte Anwendung von Röntgendiagnostik und Druckwellentherapie gewünscht. Hierzu sind kombinierte Anlagen bekannt, die Röntgenanlagen, auch in der Abbildungsrichtung verstellbare Röntgenanlagen mit Druckwellentherapiegeräten, insbesondere Stoßwellenlithotriptern, kombinieren.

Die WO 2004/006786 zeigt eine Vorrichtung zur kombinierten Anwendung von Röntgendiagnostik und Druckwellentherapie nach dem Stand der Technik. Hier sind bereits die Merkmale des Oberbegriffs des Anspruchs 1 offenbart. Allerdings ist hier neben der Röntgenanlage und seitlich neben einem dem Abdominalbereich des Patienten entsprechenden Patientenliegenbereich das Druckwellentherapiegerät angeordnet, das bei diesem Stand der Technik unabhängig von der Röntgenanlage ausgebildet ist. Das Dokument beschreibt im Einzelnen eine messtechnische Erfassung der räumlichen Relativbeziehungen zwischen beiden Geräten zur Ermöglichung der Ortung und Navigation bei der Lithotripsie mithilfe der Röntgenanlage. Hierzu ist eine direkte räumliche Nachbarschaft zwischen beiden Geräten notwendig, um Störungen der Messungen durch Personal zu verhindern.

Der vorliegenden Erfindung liegt davon ausgehend das Problem zugrunde, eine hinsichtlich der Gebrauchseigenschaften verbesserte Vorrichtung dieses Typs anzugeben.

Dieses Problem wird gelöst durch eine Vorrichtung zur Druckwellentherapie am menschlichen oder tierischen Körper gemäß Anspruch 1.

Daneben richtet sich die Erfindung auch auf vorteilhafte Verwendungen dieser Vorrichtung gemäß Anspruch 16 und bevorzugte Ausgestaltungen gemäß den abhängigen Ansprüchen. In der folgenden Beschreibung wird nicht mehr explizit zwischen der Vorrichtungskategorie und der Verwendungskategorie unterschieden, so dass die Beschreibung grundsätzlich für beide Anspruchkategorien relevant ist.

Die Grundidee der Erfindung liegt darin, die räumlichen Gegebenheiten beim Einsatz einer kombinierten Druckwellentherapie- und Röntgenvorrichtung zu verbessern. Dabei ist vor allem der Zugang zum Patienten von Interesse, und zwar bei dieser Erfindung zum Abdominalbereich, also Bauchbereich und Unterleib. Konventionelle Vorrichtungen weisen einen Ständer direkt neben der Patientenliege auf, der die Röntgenanlage an Armen trägt. Dieser Ständer steht an einer Seite neben der Patientenliegenmitte und versperrt damit den Zugang zu dem Abdominalbereich von dieser Seite. In vielen Fällen ist aber ein Zugang von beiden Seiten von Vorteil. Neben der besseren Zugänglichkeit durch eine Mehrzahl von Personen, etwa Arzt und Krankenschwester oder Pfleger, können auch seitliche Bereiche des Patienten auf beiden Seiten behandlungsbedürftig sein, beispielsweise beide Nieren. In diesen Fällen möchte der Arzt insbesondere bei Unterstützung durch einen äußeren Nierenzugang gerne direkt an der gerade behandelten Seite stehen. Daher wird der Patient bei der Verwendung von konventionellen Vorrichtungen beim Wechsel der behandelten Seite umgelagert.

Erfindungsgemäß kann eine außermittige Anordnung des Ständers erreicht werden, also neben der Patientenliege und auch an einer ihrer Längsseiten, jedoch nicht neben dem Abdominalbereich. In Betracht kommt also eine Anordnung neben dem Kopfbereich oder neben dem Fußbereich.

Konkretisiert wird dies durch die Angabe des Winkels zwischen zwei charakteristischen Linien. Die eine Linie ist eine in der Horizontalen, also der vertikalen Projektion, gedachte Verbindungslinie zwischen dem Ständer und der Röntgenquelle und der Röntgenbildaufnahmeeinrichtung bei vertikaler Durchstrahlrichtung der Röntgenanlage. Diese Linie steht für die Längserstreckung des Röntgenanlagenaufbaus an dem Ständer. Die andere Linie ist die Längsrichtung der Patientenliege, also vom Kopfbereich zum Fußbereich bzw. umgekehrt. Zwischen beiden Linien soll erfindungsgemäß ein Winkel von höchstens 65° bestehen, wohingegen der Stand der Technik hier einen Winkel von 90° vorsieht. Da von einer vertikalen Ausrichtung der Röntgenanlage bei Durchstrahlung des Abdominalbereichs ausgegangen wird, bedeutet diese Winkelangabe eine Quantifizierung der erfindungsgemäßen exzentrischen Anordnung. Besonders bevorzugt sind Winkel von höchstens 60°.

Das Personal kann nun den Abdominalbereich bei der Behandlung von beiden Seiten erreichen. Bei einer Anordnung des Ständers neben dem Kopfbereich bleiben noch die Fußseite der Behandlungsliege und die entgegengesetzte Seite der Behandlungsliege im Kopfbereich frei, insbesondere für die Anästhesie, die ja einen Kopfzugang benötigt Bei einer demgegenüber noch günstigeren Anordnung neben dem Fußbereich bleibt der Röntgenquellenbereich von drei Seiten zugänglich, jedenfalls was die Rontgenanlage betrifft.

Wichtig ist, dass die erfindungsgemäße Anordnung für die kombinierte Vorrichtung lediglich eine Eignung bedeutet. Die Vorrichtung kann durchaus auch dazu geeignet sein, abweichend aufgebaut oder verwendet zu werden. Die konventionellen Vorrichtungen andererseits sind für die erfindungsgemäße Anordnung nicht geeignet.

Bevorzugt richtet sich die Erfindung zusätzlich auf die Verwendung der erfindungsgemäßen Vorrichtung gemäß Anspruch 16, bei der die entsprechende Anordnung vorliegt.

Bei der erfindungsgemäßen Vorrichtung kann die Patientenliege verfahrbar sein und/oder der Ständer verfahrbar sein, um im Einzelfall eine optimale Geometrie zu finden. Insbesondere können diese Bestandteile auch verfahrbar sein, um sie z. B. von Zimmer zu Zimmer bewegen zu können. Beispielsweise könnte eine Mehrzahl von Patientenliegen Verwendung finden, wobei die Patienten auf außerhalb des Bereichs der Röntgenanlage und des Druckwellentherapiegeräts befindlichen Patientenliegen vorbereitet und auf diesen zur Behandlung gefahren werden, wenn andere Patienten auf anderen Patientenliegen fertig behandelt sind. Auch könnte der Ständer mit Röntgenanlage und Druckwellentherapiegerät von Raum zu Raum bewegt werden, um in verschiedenen Räumen oder auch verschiedenen Stationen oder sogar, bei einem Fahrzeugtransport, in verschiedenen Krankenhäusern oder Arztpraxen eingesetzt zu werden.

Bei einer bevorzugten Ausgestaltung ist die Röntgenanlage in ihrer Abbildungsrichtung verstellbar, und zwar durch ein Verschwenken eines Bogens, der aus den Armen gebildet ist, an denen die Röntgenquelle und die Bildaufnahmeeinrichtung montiert sind. Dadurch wird, wieder in vertikaler Projektion betrachtet, entweder die Röntgenquelle oder die Bildaufnahmeeinrichtung näher zum Ständer und das andere Teil davon weg bewegt. Bevorzugt ist hierbei eine Drehbewegung um eine horizontale Achse, die also senkrecht zu der beschriebenen Verbindungslinie zwischen Röntgenquelle und Bildaufnahmeeinrichtung einerseits und Ständer andererseits in der vertikalen Projektion liegt. Allerdings kann die Schwenkbewegung auch anders ausgeführt sein, also nicht mit fester Drehachse. Bei den bevorzugten Varianten mit einer zeitlich konstanten Drehachse läuft diese vorzugsweise durch den Fokusbereich des Druckwellengeräts.

In dieser Ausgestaltung kann also die Röntgenabbildungsrichtung aus der genau vertikalen Lage verkippt werden. Durch die beschriebene Verstellmöglichkeit kann eine für die Druckwellentherapie relevante Region im Abdominalbereich geortet werden. Somit ermöglicht die Erfindung bei dieser Ausgestaltung auch eine reine Röntgenortung und Röntgennavigation.

Eine weitere bevorzugte Ausgestaltung sieht eine zusätzliche Achse der Röntgenanlage vor, die dadurch realisiert ist, dass der Bogen verdrehbar an dem Ständer montiert ist. Erfindungsgemäß liegt diese Achse vorzugsweise nicht horizontal, sondern schräg. Bevorzugt ist ein Winkelbereich zwischen dieser Drehachse und der Horizontalen zwischen 30° und 60°, insbesondere zwischen 40° und 50°. Da Rotationen um die Röntgenabbildungsachse selbst nicht sinnvoll sind, sind mit diesen zwei rotatorischen Freiheitsgraden im Prinzip beliebige Winkelpositionen einstellbar, jedenfalls soweit es die baulichen Verhältnisse, insbesondere der Platz über und unter der Patientenliege und die Ausdehnung der Röntgenquelle und der Röntgenbildaufnahmeeinrichtung, zulassen. Insbesondere können laterale Verkippungen der Röntgenstrahlachse, also um die Patientenlängsachse verdreht, cranio-caudale Verkippungen, also um eine horizontale Achse quer zum Patienten verdreht, und Strahlrichtungsumkehrungen realisiert werden. Die cranio-caudale Position wird dabei i. d. R. nur angenähert erreicht, aber dennoch so bezeichnet.

Bei weiteren bevorzugten Ausgestaltungen ist auch das Druckwellentherapiegerät beweglich. Vorzugsweise ist es an dem Bogen montiert und entlang dem Bogen verfahrbar, wobei diese Ausgestaltung wiederum vorzugsweise isozentrisch ist. Der Mittelpunkt des Verfahrweges des Druckwellentherapiegeräts, der Schnittpunkt aller möglichen Röntgenstrahlachsen und der Fokusbereich des Druckwellentherapiegeräts fallen also vorzugsweise zusammen.

Darüber hinaus ist es günstig, wenn das Druckwellentherapiegerät eine Parkposition und eine Arbeitsposition einnehmen kann. Bei der Arbeitsposition liegt der Fokusbereich im Röntgenstrahlengang. In der Parkposition befindet sich das Druckwellentherapiegerät ganz außerhalb des Röntgenstrahlengangs, soweit dieser für die Abbildung genutzt wird, um möglichst wenig zu stören. Idealerweise ist der Bewegungsmechanismus für die Bewegungen zwischen diesen beiden Positionen ebenfalls entlang dem Bogen verfahrbar und funktioniert in den verschiedenen Schwenkpositionen des Druckwellentherapiegeräts. Damit kann das Druckwellentherapiegerät während des Schwenkvorgangs aus dem Liegenbereich herausgezogen sein und in bestimmen therapeutisch relevanten Positionen, etwa unter der Liege mit vertikaler Beaufschlagungsrichtung nach oben, lateral neben dem Patienten knapp über der Liege mit horizontaler Beaufschlagungsrichtung und über der Liege mit vertikaler Beaufschlagungsrichtung nach unten, in eine jeweilige Arbeitsposition verfahrbar sein.

Zudem ist es von Vorteil, laterale Verstellmöglichkeiten vorzusehen, um, insbesondere bei der bevorzugten isozentrischen Ausführung, die interessierende Region in dem Abdominalbereich in den von der Röntgenanlage und dem Druckwellentherapiegerät erfassten Bereich zu bringen. Dabei können zwei horizontale Verstellrichtungen vorgesehen sein. Letztlich kommt es auf die Relativanordnung zwischen dem Ständer und der Patientenliege an, so dass die Patientenliege und/oder der Ständer verstellt werden können. Bevorzugt ist eine Ständerverstellung, weil sich damit keine Erschütterungen für den Patienten ergeben und nicht auf etwaige von ihm ausgehende Leitungen in Zusammenhang mit Kathetern, der Anästhesie oder anderem, geachtet werden muss. Mit einer Ständerverstellung ist dabei nicht unbedingt ein Verfahren des Ständers insgesamt über den Boden gemeint. Es genügt für die Ständerverstellung, wenn die gemeinsame Konstruktion von Druckwellentherapiegerät und Röntgenanlage, also insbesondere der Bogen, verfahren wird. Diese Verstellmöglichkeit besteht vorzugsweise auch vertikal.

Wenn das Druckwellentherapiegerät jedenfalls in der Ebene des Röntgenbildes bereits auf einen bzgl. der Röntgenbilder festgelegten Ort fokussiert ist, etwa die Mitte des Röntgenbildes, kann durch die beschriebene Relativeinstellung zwischen Ständer und der Patientenliege das Druckwellentherapiegerät insoweit schon auf die zu behandelnde Region eingestellt werden. I. d. R. ist dann eine zusätzliche Einstellung in der Richtung der Röntgenaufnahmeeinrichtung notwendig. Für die Bestimmung der Höheneinstellung des Therapiegebietes soll bei reiner Röntgenortung eine weitere Durchstrahlungsrichtung, beispielsweise verschieden von der vertikalen Richtung, bevorzugt unter 30° zur Vertikalen, einstellbar sein. Dies kann etwa erreicht werden durch eine Drehung des C-Bogens. Die Einstellung selbst kann ebenfalls durch eine Einstellung des Druckwellentherapiegeräts (incl. Röntgeneinrichtung)oder auch durch ein entsprechendes Nachjustieren, etwa eine Höhenanpassung, der Patientenliege erfolgen. Bevorzugt wird die Höheneinstellung der Liege, weil damit auch eine günstige Höheneinstellung für den Aufstieg des Patienten erreicht werden kann.

Schließlich ist eine hohle Ausführung des Druckwellentherapiegeräts bevorzugt. Dies gilt insbesondere für Stoßwellenlithotripter. Die Druckwellenquelle kann dabei eine Hohlspule sein. Jedenfalls soll ein Durchtritt der Röntgenstrahlen durch das Druckwellentherapiegerät möglich sein, vorzugsweise zumindest axial. Damit können die Röntgenanlage und das Druckwellentherapiegerät bei jeweils vertikaler Arbeitsrichtung gleichzeitig betrieben werden. Hierbei ist es übrigens günstig, bei der geschilderten Verstellbarkeit das Druckwellentherapiegerät in der Nähe der Röntgenquelle anzuordnen, weil damit die durch die Hohlgeometrie evtl. unvermeidliche Einschränkung des Bildquerschnitts der Röntgenanlage geringer ist als bei einer Anordnung in der Nähe der Bildaufnahmeeinrichtung, beispielsweise des Bildverstärkers.

Vorzugsweise existiert sogar eine weitere, gegenüber der axialen Richtung etwas geneigte mögliche Durchtrittsrichtung für Röntgenstrahlen durch das Druckwellentherapiegerät, vorzugsweise für eine lateral verkippte zweite Röntgenabbildung bei einer Anordnung des Druckwellentherapiegeräts in seiner Arbeitsposition.

Die laterale Anordnung des Druckwellentherapiegeräts stört die Röntgenabbildung übrigens i. d. R. nicht, jedenfalls dann nicht, wenn diese nahe der vertikalen Abbildungsachse oder auch mit einer cranio-caudalen Position erfolgt.

Der Ständer kann einen Fuß mit in der Richtung der Verbindungslinie zwischen Röntgenquelle und Bilderzeugungseinrichtung einerseits und Ständer andererseits etwas größerer Ausdehnung aufweisen, um den Schwerpunkt der Gesamtkonstruktion abstützen zu können. Dieser Fuß kann mit seiner Verkleidung ein nahes Heranrücken an die Patientenliege erschweren, weil er mit Stützkonstruktionen der Patientenliege kollidiert oder jedenfalls den möglicherweise für andere Zwecke benötigten Raum unter dem Patienten einschränkt. In diesem Zusammenhang ist der Ständer vorzugsweise in dem relevanten Bereich, also dem der Röntgenquelle und der Röntgenbildaufnahmeeinrichtung zugewandten Bereich, angeschrägt ausgestaltet. Damit kann eine sinnvolle Abstützung mit einer in dem problematischen Bereich möglichst schlanken Bauform kombiniert werden. Die Anschrägung der Verkleidung soll dabei zumindest die Hälfte der der Röntgenquelle und der Röntgenbildaufnahmeeinrichtung zugewandten Seite umfassen, der nicht angeschrägte Bereich soll also höchstens die Hälfte ausmachen. Bevorzugt ist ein nicht angeschrägter Rest mittig vorgesehen und sind zwei Anschrägungen an beiden Seiten davon vorgesehen. Die Anschrägungen müssen nicht gerade Flanken haben, sollen aber jedenfalls auf schräge Winkel im Bereich zwischen 10° und 60°, vorzugsweise mindestens 15° und vorzugsweise höchstens 45°, hin angeschrägt sein. Seitlich von einem nicht angeschrägten Bereich von höchstens der Hälfte der betrachteten Verkleidungsfront sollen also entsprechende Winkel erreicht werden. Damit kann der Ständer in der erfindungsgemäßen schrägen Orientierung recht nah an die Patientenliege herangerückt werden, ohne wesentlich oder überhaupt unter die Patientenliege zu ragen und ohne die Bewegungsfreiheit (besonders im Fußbereich) des Behandlers, der seitlich an der Liege steht, zu behindern. Bei einer doppelseitigen Anschrägung gilt dies auch in verschiedenen Anordnungen, also rechts oder links von der Patientenliege bzw. neben dem Kopf- oder dem Fußbereich. Zur Veranschaulichung wird auf das Ausführungsbeispiel verwiesen.

Eine weitere bevorzugte Ausgestaltung des Ständers weist ein Geräteregal für weitere medizintechnische Geräte auf, etwa für Endoskopiegeräte oder Steuerungs- und

Auswertegeräte, eine Ultraschalldiagnostikeinheit u. Ä. Dieses Geräteregal (oder "Rack") ist in dem Sinn "oben" angeordnet, als es zumindest teilweise höher als die Patientenliege, d. h. die Patientenebene, montiert ist. Es kann damit aus dem Fußbereich herausgehalten werden, der möglicherweise für andere Dinge benötigt wird und leichter zugänglich und damit einfacher zu bedienen oder zu reinigen ist. Schließlich ist diese Anordnung ergonomisch günstiger zur Bedienung der Geräte als eine Anordnung unter Hüfthöhe. Bei einer besonders bevorzugten Ausgestaltung ist das Geräteregal auf dem eigentlichen Ständer angeordnet, wie das Ausführungsbeispiel zeigt.

Die Patientenliege weist bevorzugt eine exzentrische Säule auf, um den Bereich unterhalb des Abdominalbereichs des Patienten freizulassen. Damit kann beispielsweise ein Teil der Röntgenanlage unter den Abdominalbereich geschoben werden. Zusätzlich weist die Patientenliege einen von der exzentrischen Säule ausgehenden Stützfuß auf, der sich relativ flach auf dem Boden bis mindestens in den Abdominalbereich erstreckt, um eine ausreichende Stabilität der Patientenliege sicherzustellen. Diese Ausgestaltung eignet sich besonders auch für verfahrbare Liegen, die nicht fest auf einem Boden verschraubt sind. Auch hier wird zur Veranschaulichung auf das Ausführungsbeispiel verwiesen.

Die Erfindung wird anhand eines Ausführungsbeispiels näher erläutert, wobei die einzelnen Merkmale auch in anderen Kombinationen erfindungswesentlich sein können und sich auf die Vorrichtungskategorie und die Verwendungskategorie beziehen.

Es zeigen:
- Figur 1a + b: zwei Draufsichten auf erfindungsgemäße Vorrichtungen in verschie- dener Anordnung,
- Figur 2: eine Aufrissansicht der Vorrichtung aus Figur 1b ohne Druckwellen- therapiegerät in Patientenliegenlängsrichtung gesehen,
- Figur 3: eine Seitenansicht der Vorrichtung aus Figur 2 mit gegenüber Figur 2 umgekehrter Durchstrahlungsrichtung der Röntgenanlage und wieder ohne Druckwellentherapiegerät dargestellt.
- Figur 4a - c: Aufrissdarstellungen entsprechend Figur 2, jedoch mit Druckwellen- therapiegerät in verschiedenen Positionen,
- Figuren 5 - 10: Seitenansichten des Ständers mit verschiedenen Positionen des C- Bogens,
- Figuren 11 - 14: Draufsichten entsprechend Figur 1b mit verschiedenen C-Bogen- Positionen.

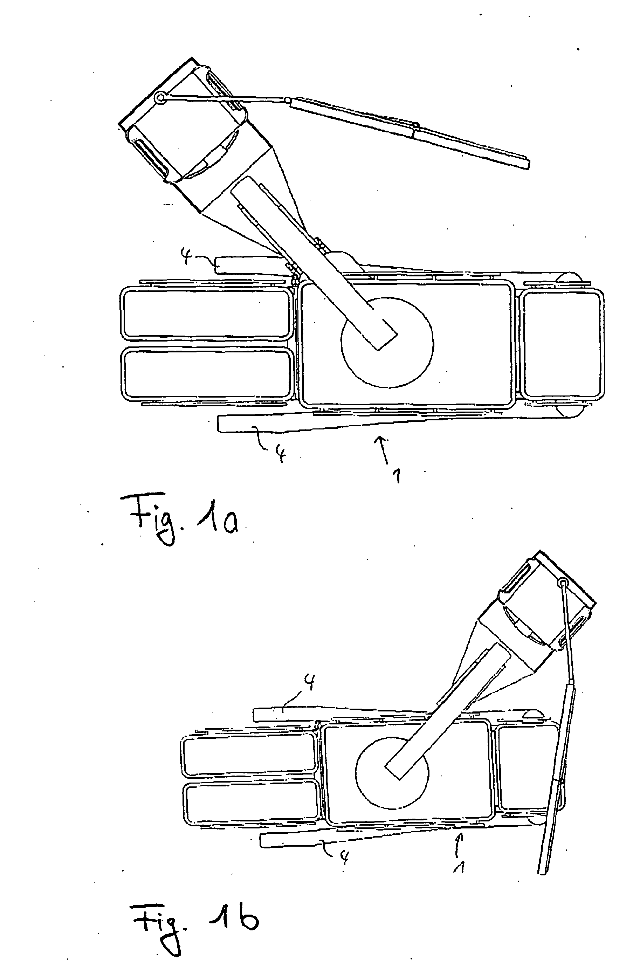

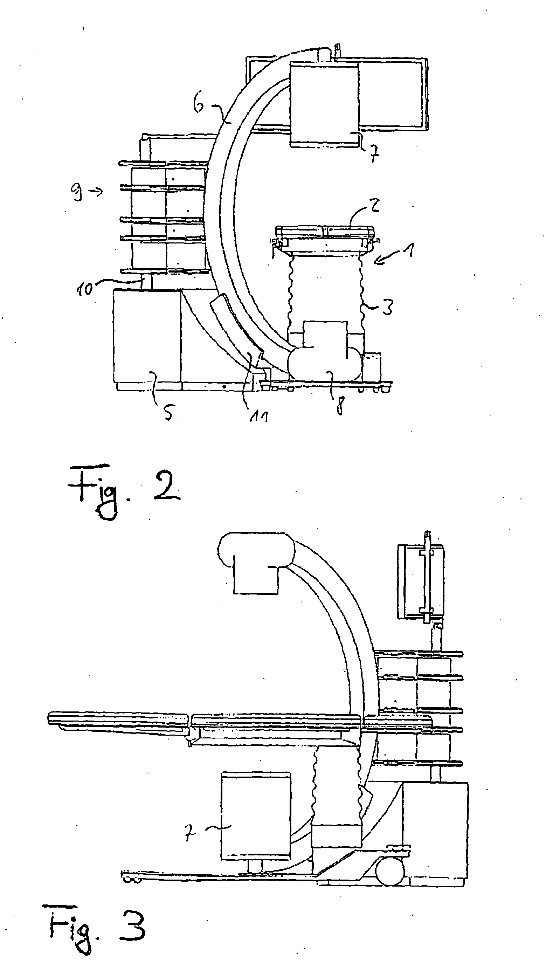

In den Figuren 1a, b und 2 erkennt man zunächst eine Patientenliege 1 mit einer in eine Kopfauflage, eine Oberkörperauflage und zwei Beinauflagen gegliederten Liegeplatte 2. Die Liegeplatte 2 ist auf einem mit einer Balgenabdeckung verkleideten Säule 3 montiert, die unter dem Kopfteil und dem benachbarten Bereich des Oberkörperteils der Liegeplatte 2 liegt, wie die später noch näher beschriebene Figur 3 zeigt.

Die Säule 3 stützt sich mit zwei im Wesentlichen zur Längsrichtung der Liege (in Figur 1 horizontal in der Zeichenebene) parallelen Längsfüßen 4 auf dem Boden ab und ist, wie in Figur 2 ansatzweise erkennbar, auf diesen Füßen über Rollen verfahrbar.

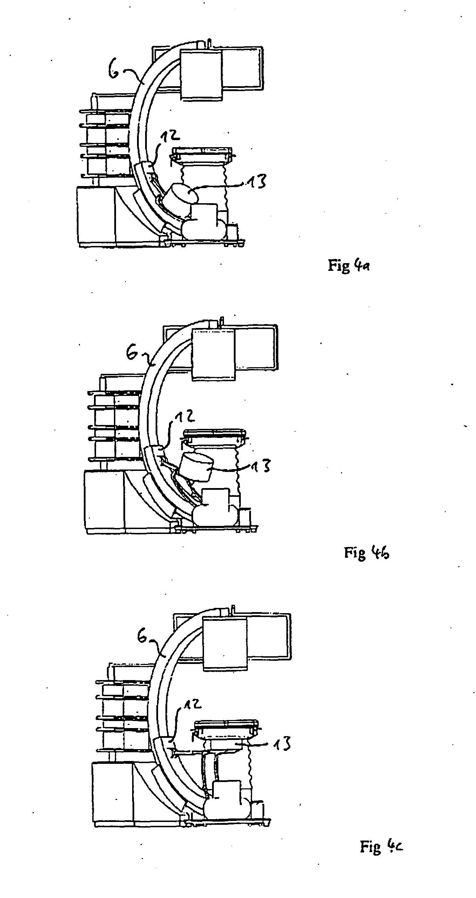

Neben der Patientenliege 1 - 4 steht auf dem Boden ein Ständer 5, der einen im Wesentlichen halbkreisförmigen C-Bogen 6 trägt. An den Enden des C-Bogens 6 befindet sich in den Figuren 1a, b und 2 oben ein Röntgenbildverstärker 7 als Bildaufnahmeeinrichtung und unten eine Röntgenquelle 8. Die Röntgenquelle 8 liegt unter der Liegeplatte 2 der Patientenliege 1 und neben der Säule 3, vgl. Figur 4. Die zwischen der Röntgenquelle 8 und dem Bildverstärker 7 verlaufende Röntgenstrahlrichtung verläuft vertikal durch den mittleren bis unteren Bereich der Oberkörperauflage der Liegeplatte 2, also den Abdominalbereich mittig in der Patientenliege 1.

Die Figuren 1a und b zeigen deutlich, dass der Ständer 5 exzentrisch neben der Patientenliege 1 steht, und zwar in Figur 1a neben dem Fußbereich und in Figur 1b neben dem Kopfbereich. Dabei verdeutlicht die vertikale Draufsicht auf den C-Bogen 6 die bereits mehrfach erwähnte Bezugslinie, nämlich die Verbindungslinie zwischen Ständer 5 und Röntgenquelle 8 bzw. Bildverstärker 7, die hier unter 45° zur Patientenliegenlängsrichtung, nämlich der Horizontalen in der Zeichenebene der Figur 1, verläuft. Damit ist der Abdominalbereich der Patientenliege 1 von beiden Seiten frei zugänglich. Gleiches gilt auch für den Kopfbereich in Figur 1a und dem Beinbereich in Figur 1b. Man erkennt schließlich (vgl. auch Figur 2 und 3), dass der der Patientenliege 1 zugewandte Bereich des Ständers 5 angeschrägt ist, wobei die schrägen Flanken zu der bereits erwähnten Verbindungslinie einen Winkel von etwa 20° bilden und in einen im Verhältnis zur Breite des Ständers 5 schmalen Fuß auslaufen, der den Ständer 5 zur Patientenliege 1 hin abstützt. Der Fuß ist dabei nur etwa ein Viertel so breit wie der Ständer 5 selbst.

Figur 2 zeigt ein auf dem Ständer 5 aufgebautes Geräteregal 9 mit schwenk- oder drehbaren Regalplatten für verschiedenste Geräte, beispielsweise Endoskopiegeräte, Bediengeräte für Videokameras, Pumpen oder auch einen sog. Steinlaser zur laserbasierten Steinzerstörung. Dieses Geräteregal 9 ist auf einer Tragestange 10 auf dem Ständer 5 aufgebaut, liegt damit in der Vertikalprojektion der Figur 1 erkennbar über dem Ständer 5 und zu etwa zwei Dritteln über der Ebene der Patientenliegeplatte 2. Es ist damit ergonomisch günstig untergebracht, aus dem Fußbodenbereich entfernt, was Platz spart und die Reinigungsarbeiten erleichtert und im Übrigen die Geräte schützt, und in einer praktischen Weise fest mit dem Ständer 5 verbunden. Dieser ist nämlich ebenfalls verfahrbar und kann mit dem C-Bogen 6 und dem Geräteregal 9 in andere Positionen in demselben Raum oder auch in einen anderen Raum gefahren werden. An der Arbeitsposition wird der Ständer 5 sicher auf dem Boden fixiert, z. B. durch Absenken.

Die Tragestange 10 läuft durch das Geräteregal 9 hindurch und oben darüber hinaus und trägt an einem Hebelarm zwei große über Berührung bedienbare Flachbildschirme zur Darstellung von Röntgenbildern oder auch Bildern der Videokamera, also aus der Endoskopie, von für den Benutzer wesentlichen Daten, von Ultraschallbildem, etc.

Man erkennt in Figur 2, dass der C-Bogen 6 abweichend von Figur 1 nicht neben dem Fußbereich, sondern neben dem Kopfbereich der Patientenliege 1 angeordnet ist, wieder unter 45° zur Patientenliegenlängsrichtung. Diese Anordnung ist ebenfalls erfindungsgemäß, bietet hinsichtlich der Zugänglichkeit des Abdominalbereichs gleiche Vorteile, schafft aber nicht die gleiche gute Zugänglichkeit des Kopfbereichs wie die Anordnung aus Figur 1. Stattdessen ist die Zugänglichkeit des Beinbereichs verbessert. Insbesondere könnte (bei einer nicht gezeichneten offenen Gestaltung der Patientenliege 1 im Beinbereich) der Genitalbereich und insbesondere der Harnleiter zwischen den Beinen des Patienten besser zugänglich sein, etwa wenn von dort aus Endoskope oder Katheter eingeführt werden sollen.

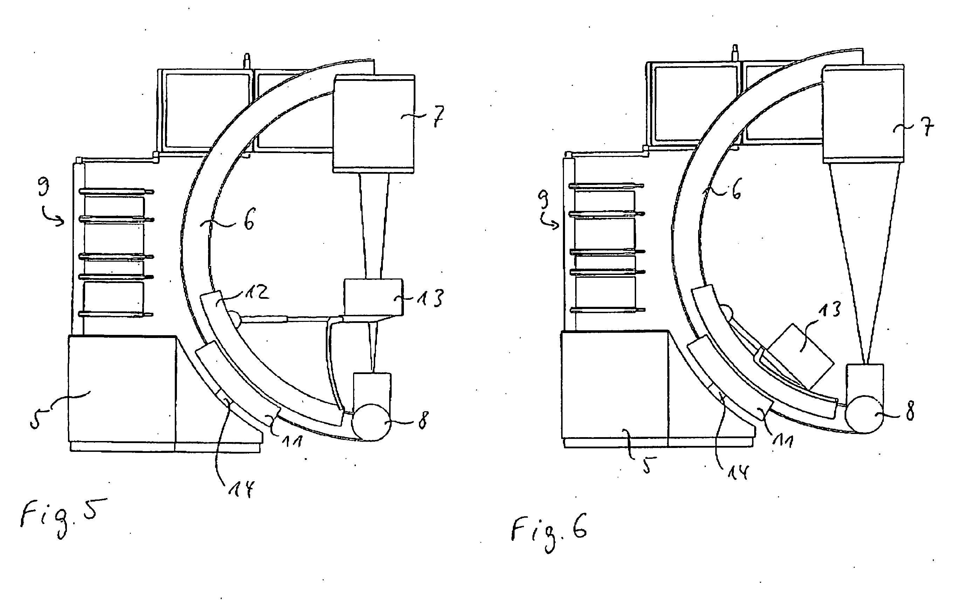

Man erkennt in Figur 2, dass der C-Bogen 6 in einer schuhähnlichen Führung 11 an dem Ständer 5 gehalten ist. In dieser schuhähnlichen Führung kann der C-Bogen entlang seiner Bogenform verfahren werden, was einer Drehung um eine horizontale Achse entspricht, die zu der Vertikalprojektion des C-Bogens 6 aus Figur 1 senkrecht und durch den Abdominalbereich des Patienten sowie durch das Röntgenstrahlbündel verläuft. Hierzu wird auf die Figuren 5 ff verwiesen.

In der in Figur 2 erkennbaren und bereits früher erwähnten Säule 3 ist eine linear arbeitende Hubeinrichtung vorgesehen, mit der die vertikale Höhe der Liegeplatte 2 über dem Boden eingestellt werden kann, um den Patienten vertikal zu positionieren. Zusätzlich könnten auch ein oder zwei lateral wirkende Lineartriebe in der Säule 3 vorgesehen sein, um den Patienten zwei- oder dreidimensional positionieren zu können. Bei dem vorliegenden Ausführungsbeispiel sind allerdings die beiden lateralen Freiheitsgrade in dem Ständer 5 realisiert, der den C-Bogen 6 trägt. Der C-Bogen 6 ist also durch zwei horizontal wirkende Lineartriebe zweidimensional verstellbar und die Liegeplatte 2, wie erwähnt, vertikal. Damit kann der Patient relativ zu dem C-Bogen 6 oder umgekehrt der C-Bogen 6 relativ zu dem Patienten dreidimensional positioniert werden.

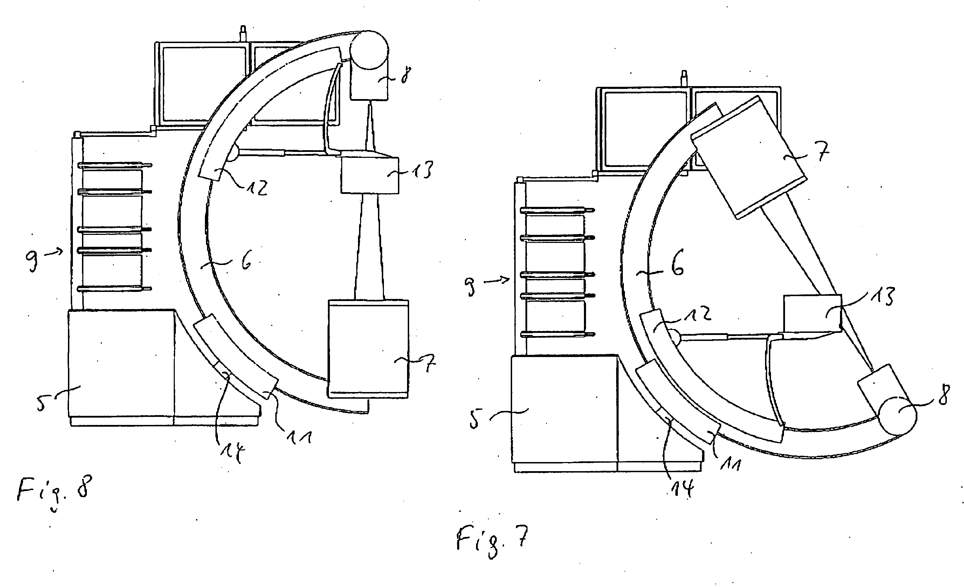

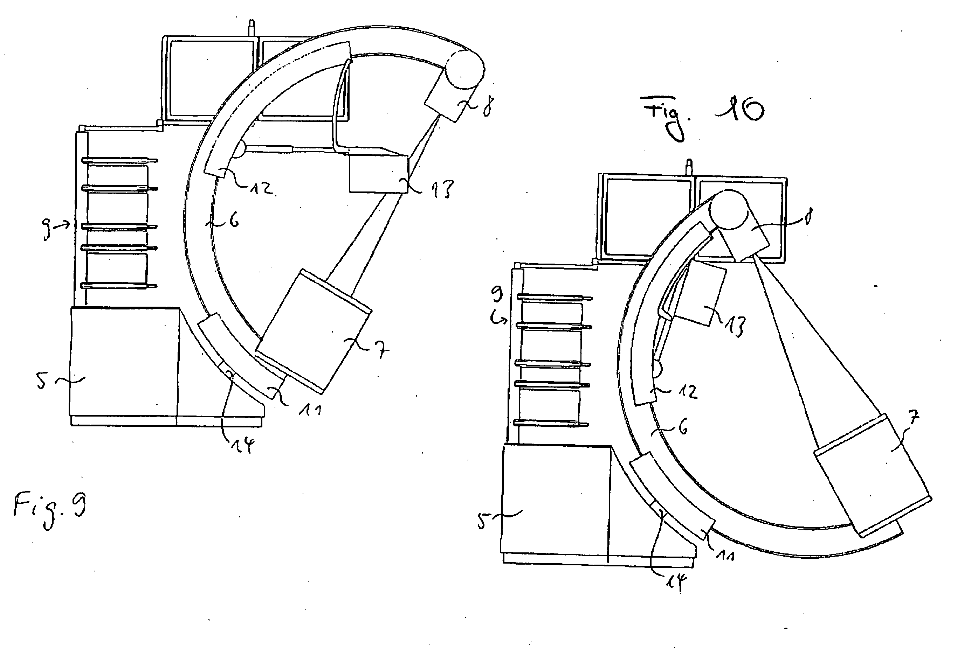

Figur 3 zeigt die Situation aus Figur 2 mit umgekehrter Anordnung der Röntgenanordnung, also der Röntgenquelle 8 und des Bildverstärkers 7. Diese Anordnung ist in manchen Fällen günstig, etwa wenn der Arzt über dem Patienten relativ viel Platz benötigt und ihn die im Vergleich zu dem Bildverstärker 7 kleinere Röntgenquelle 8 weniger behindert. Außerdem kann der Stoßwellenlithotripter, der in Figur 3 nicht dargestellt ist, über dem Patienten angeordnet werden und befindet sich dann näher an der Röntgenquelle 8, um in dieser Weise weniger abschattend beim Durchtritt der Röntgenstrahlen durch die Hohlgeometrie zu wirken, vgl. Figur 8 und 9.

Die Anordnung in Figur 2 wiederum ist aus gleichen Gründen in Fällen bevorzugt, in denen der Stoßwellenlithotripter unter der Patientenliegeplatte 2 angeordnet ist. Außerdem ist die Anordnung in Figur 2 strahfenschutztechnisch günstiger, weil die Röntgenquelle 8 unter der Platte stärker durch die Liegeplatte und evtl. weitere nicht gezeichnete Vorrichtungen abgeschirmt wird.

Die Figuren 4a - c entsprechen Figur 2, zeigen aber zusätzlich einen auf dem C-Bogen 6 entlang diesem verfahrbaren weiteren Führungsschuh 12. Dieser liegt auf den C-Bogen 6 bezogen radial innen und nicht, wie die schuhähnliche Führung 11 des C-Bogens 6 selbst, radial außen. Der Führungsschuh 12 trägt an zwei Gelenkachsen ein in den Figuren 4a - c in seiner Bewegung dargestelltes Hebelgestänge, an dem ein in der Figur der Einfachheit halber zylindrisch dargestellter Stoßwellenlithotripter 13 angebracht ist. Der Lithotripter 13 kann in die in Figur 4a dargestellte Parkposition bewegt werden, in der er soweit möglich an den C-Bogen 6 angenähert ist. Aus dieser Parkposition kann er gemäß den Figuren 4b und c in eine Behandlungsposition in Figur 4c gebracht werden, in der er hier von unten in eine entsprechende Lücke der Liegeplatte 2 eingreift und maximal an den Patienten angenähert ist. In der Behandlungsposition liegt er grundsätzlich isozentrisch zu der Röntgenanlage 7. 8. Im Übrigen nimmt der Stoßwellenlithotripter 13 durch seine Montage an dem C-Bogen 6 an der bereits erwähnten dreidimensionalen Positionierung zwischen Patient und C-Bogen 6 teil.

Figur 5 zeigt eine seitliche Darstellung des Ständers 5 mit Geräteregal 9, C-Bogen 6 und Röntgenanlage 7, 8. Die Patientenliege 1 ist hier der Einfachheit halber weggelassen, die Blickrichtung ist im Unterschied zu Figur 4a - c genau seitlich, so dass also der C-Bogen 6 parallel zur Zeichenebene läuft. Hier ist eine Drehachse 14 erkennbar, um die die Führung 11 und der C-Bogen 6 gedreht werden können und die unter 45° zur Horizontalen liegt.

Figur 5 zeigt ferner symbolisch die aus der Röntgenquelle 8 austretenden Röntgenstrahlen, die durch den Lithotripter, d. h. durch eine zentrale Öffnung darin, hindurchtreten, um den Bildverstärker 7 zu erreichen. Diese koaxiale Anordnung des Lithotripters 13 und der Röntgenanlage 7, 8 ist an sich bekannt und basiert auf einer Hohlspulentechnologie des Lithotripters 13.

Figur 6 zeigt im Vergleich dazu den Lithotripter 13 in der Parkposition und den dabei größeren Öffnungswinkel der Röntgenstrahlen, also den größeren erfassten Bildbereich und das größere Bildformat. Dieser grundsätzliche Zusammenhang der Einschwenkbarkeit des Lithotripters 13 in den Röntgenstrahlengang und der Begrenzung des Bildbereichs gilt im Prinzip auch für andere Positionen des C-Bogens 6, beispielsweise in Figur 8.

Figur 7 entspricht Figur 5, jedoch wurde der C-Bogen 6 um eine auf der Zeichenebene senkrechte und durch den Fokuspunkt des Lithotripters 13 laufende Achse verschwenkt. Dabei wurde der C-Bogen 6 in der Führung 11 verschoben und diese Verschiebung durch ein Verfahren des Führungsschuhs 12 kompensiert. Die Position des Lithotripters 13 ist also gegenüber der Figur 5 unverändert. Der Schwenkwinkel beträgt 30°. Unter diesem Winkel zeigt der Lithotripter 13 eine zweite Durchtrittsöffnung für die Röntgenstrahlen, die wiederum den Öffnungswinkel der Röntgenstrahlen etwas beschränkt, wie figürlich dargestellt. Die erste Richtung geht durch die zylindrische Quellenspule, die zweite unter 30° gekippt durch die Quelle, aber an der Spule vorbei. Wenn der Lithotripter 13 in die Parkposition entsprechend Figur 6 bewegt wird, besteht diese Einschränkung nicht. Jedenfalls lässt die gezeichnete C-Bogenposition eine laterale Röntgenabbildung zu.

Figur 8 entspricht Figur 5, jedoch sind die Röntgenanlage 7, 8 und der Lithotripter 13 mit seinem Führungsschuh 12 "auf den Kopf gestellt". Diese Position kann erreicht werden durch ein Bewegen des Lithotripters 13 in die Parkposition gemäß Figur 6, ein Verschwenken des C-Bogens 6 in eine Figur 7 ähnelnde Position und (auch gleichzeitiges) Verdrehen des C-Bogens 6 um die Drehachse 14 um 180°, ein Zurückschwenken in eine senkrechte Anordnung der Röntgenanlage 7, 8 und dann wieder in die in Figur 8 dargestellte Behandlungsposition Bewegen des Lithotripters 13.

In dieser Position erfolgt die Stoßwellenbehandlung von oben und ist die Röntgenabbildungsrichtung gegenüber Figur 5 invertiert. Wenn in dieser Position der Lithotripter 13 in die Parkposition bewegt wird (nicht gezeigt), besteht besonders viel Platz über dem Patienten, weil die Röntgenquelle 8 kleiner und weiter vom Behandlungszentrum entfernt ist als der Bildverstärker 7.

Sowohl in der Position des C-Bogens 6 aus Figur 5 als auch in der aus Figur 8 lässt sich der Führungsschuh 12 so verfahren, dass der Lithotripter 13 seitlich an den Patienten herangebracht werden kann. Infolge der isozentrischen Konstruktion liegt dabei der Fokusbereich des Lithotripters 13 weiterhin mittig im Röntgenstrahlengang. In Figur 5 müsste der Führungsschuh 12 um 90° nach oben gefahren werden, in Figur 8 um 90° nach unten.

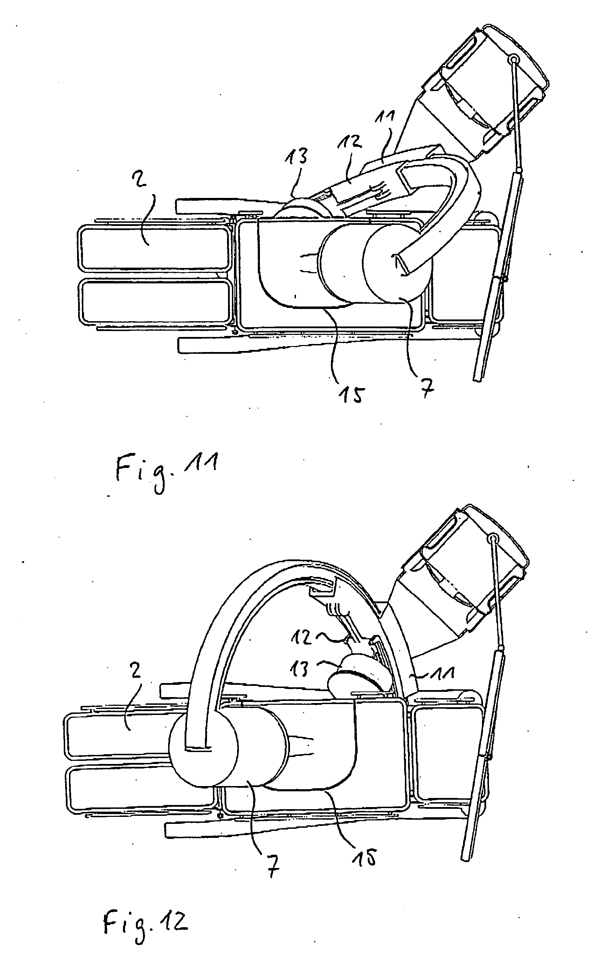

Figur 9 zeigt eine genauso gegenüber Figur 8 verschwenkte Position der Röntgenanlage 7, 8, wie die Position aus Figur 7 im Verhältnis zu der aus Figur 5. Es gelten analoge Anmerkungen. Schließlich zeigt Figur 10 ein Verschwenken der Röntgenanlage 7, 8 gegenüber der Position aus Figur 8 mit anderem Drehsinn, aber um die gleiche Schwenkachse. Hier ist der Lithotripter 13 in die Parkposition gebracht. Die Figuren 11-14 entsprechen als Draufsicht zunächst Figur 1b, wobei sie verschiedene Einstellmöglichkeiten des C-Bogens 6 zeigen. Eine erste vertikale Einstellmöglichkeit zeigt bereits Figur 1b.

In Figur 11 ist der C-Bogen 6 demgegenüber sowohl um die anhand der Figuren 7 - 10 beschriebene Achse als auch um eine weitere Drehachse bewegt worden, wobei letztere in der Figur von links unten nach rechts oben und schräg zur Papierebene läuft. Sie liegt also mit ihrer horizontalen Komponente parallel zu der bereits früher zur Erläuterung herangezogenen horizontalen Verbindungslinie zwischen einerseits der Röntgenquelle 8 und der Bildaufnahmeeinrichtung 7 und andererseits dem Ständer 3, und zwar bezogen auf die vertikale Durchstrahlung wie in Figur 1b. Die entsprechende mechanische Drehachse 14 ist in Figur 5 eingezeichnet. Die Kombination beider Drehbewegungen führt zu einer angenäherten cranio-caudalen Position, also eine aus der Vertikalen in Richtung zu der Längsachse des Patienten bewegten Durchstrahlungsrichtung.

Figur 12 zeigt eine genau umgedrehte Bewegung, wieder mit Kombination beider Drehachsen.

In den Figuren 11 - 14 ist im Übrigen zusätzlich zu Figur 1b nicht nur der Stoßwellenlithotripter 13 eingezeichnet, der bereits zuvor erläutert wurde, sondern auch ein U-förmig ausgeschnittener Bereich 15 in der Patientenliege 2 im Abdominalbereich des Patienten. Dieser Teil 15 der Patientenliege kann entnommen werden, um eine Annäherung des Lithotripters 13 an den Patienten wie in Figur 4c, Figur 5 und Figur 7 zu erreichen, und kann bei Bedarf auch wieder eingesetzt werden.

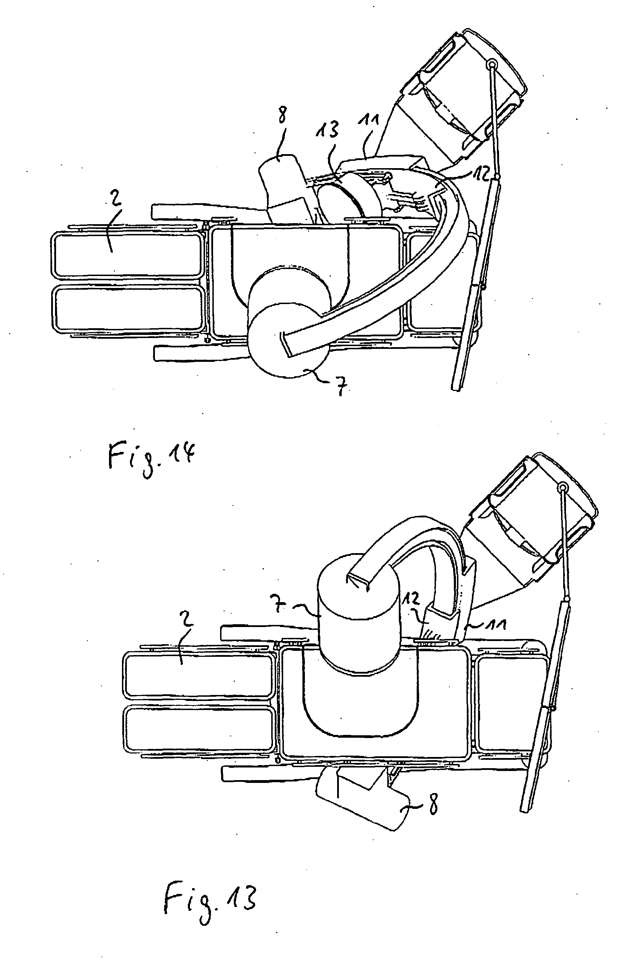

Figur 13 zeigt wieder eine Kombination beider Drehachsen, hier aber mit einer resultierenden Durchstrahlungsrichtung, die gegenüber der Vertikalen in Richtung zu der Querrichtung des Patienten bewegt ist, also eine sog. laterale Verkippung.

Das Gleiche gilt für Figur 14, aber mit umgekehrter Richtung.

Die Figuren 11 - 14 veranschaulichen die in gewissen Winkelbereichen beliebigen Kipppositionen, die sich mit der erfindungsgemäßen Vorrichtung erzielen lassen, und denen die schräge Anordnung des Ständers 5 relativ zu der Patientenliege 1 und die schräge Anordnung der Drehachse 14 relativ zur Horizontalen nicht im Wege steht.

## Patentansprüche

1. Vorrichtung zur Druckwellentherapie am menschlichen oder tierischen Körper mit
einem fokussierenden Druckwellentherapiegerät (13),
einer integrierten Röntgenanlage (7, 8), deren Röntgenquelle (8) und Bildaufnahmeeinrichtung (7) jeweils an Tragearmen (6) an einem Ständer (5) befestigt sind,
und
einer Patientenliege (1) neben dem Ständer (5),
wobei die Röntgenanlage (7, 8) und das Druckwellentherapiegerät (13) zur Erfassung eines Abdominalbereichs eines Patienten auf der Patientenliege (1) ausgelegt sind,
der Abdominalbereich mit der Röntgenanlage (7, 8) vertikal durchstrahlbar ist **dadurch gekennzeichnet, dass**
die Vorrichtung dazu ausgelegt ist, dass der Ständer (5) einerseits und die Röntgenquelle (8) und die Bildaufnahmeeinrichtung (7) der Röntgenanlage andererseits bei der vertikalen Durchstrahlung des Abdominalbereichs in vertikaler Projektion und bei in einer Behandlungsposition befindlichem Druckwellentherapiegerät (13) eine Verbindungslinie bilden, die zu der Längsachse der Patientenliege (1) einen Winkel von höchstens 65° bildet,
und dass die Vorrichtung einen dem Abdominalbereich des Patienten entsprechenden Bereich der Patientenliege (1) an beiden Seiten der Patientenliege (1) zur Zugänglichkeit durch Personen freilässt.

2. Vorrichtung nach Anspruch 1, bei der die Röntgenquelle (8) und die Bildaufnahmeeinrichtung (7) der Röntgenanlage an fest zusammenhängenden Armen (6) befestigt sind, die einen Bogen (6) bilden, der an dem Ständer (5) so verschwenkbar ist, dass sich in vertikaler Projektion eines von Röntgenquelle (8) und Bildaufnahmeeinrichtung (7) dem Ständer (5) annähert und das andere von dem Ständer (5) entfernt.

3. Vorrichtung nach Anspruch 2, bei der der Bogen (6) bei dem Verschwenken mit der Röntgenquelle (8) und der Bildaufnahmeeinrichtung (7) um eine feste horizontale Schwenkachse verschwenkbar ist, die senkrecht zu den Verbindungslinien zwischen einerseits der Röntgenquelle (8) und der Bildaufnahmeeinrichtung (7) und andererseits dem Ständer (5) bei vertikaler Durchstrahlung liegt und durch einen Fokusbereich des Druckwellentherapiegerätes (13) läuft.

4. Vorrichtung nach Anspruch 2 oder 3, bei der der Bogen (6) an dem Ständer (5) um eine Achse (14) verdrehbar montiert ist, die in vertikaler Projektion bei der vertikalen Durchstrahlung des Abdominalbereichs parallel zu den Verbindungslinien von einerseits Röntgenquelle (8) und Bildaufnahmeeinrichtung (7) und andererseits Ständer (5) bei vertikaler Durchstrahlung ist und mit der Vertikalen einen Winkel zwischen 30° und 60° bildet.

5. Vorrichtung nach einem der Ansprüche 2 - 4, bei der das Druckwellentherapiegerät (13) an dem Bogen (6) und entlang dem Bogen (6) verfahrbar montiert ist.

6. Vorrichtung nach Anspruch 5, bei der das Druckwellentherapiegerät (13) entlang dem Bogen (6) um eine horizontale Achse verschwenkbar ist, die senkrecht zu den Verbindungslinien zwischen einerseits der Röntgenquelle (8) und der Bildaufnahmeeinrichtung (7) und andererseits dem Ständer (5) bei vertikaler Durchstrahlung liegt und durch einen Fokusbereich des Druckwellentherapiegerätes (13) läuft.

7. Vorrichtung nach einem der vorstehenden Ansprüche, bei der das Druckwellentherapiegerät (13) mit einem Bewegungsmechanismus in eine Parkposition außerhalb des Röntgenstrahlengangs und in eine Arbeitsposition bewegbar ist , wobei in der Arbeitsposition der Fokusbereich des Druckwellentherapiegerätes (13) in dem Röntgenstrahlengang liegt.

8. Vorrichtung nach einem der vorstehenden Ansprüche, bei der die Relativanordnung des Ständers (5) und der Patientenliege (1) in zwei horizontalen und einer vertikalen Richtung/en verstellbar ist.

9. Vorrichtung nach einem der vorstehenden Ansprüche, bei der das Druckwellentherapiegerät (13) eine Hohlgeometrie hat, die einen axialen Durchtritt von Röntgenstrahlen erlaubt.

10. Vorrichtung nach Anspruch 9, bei der die Hohlgeometrie den Durchtritt von Röntgenstrahlen in zwei zueinander gewinkelten Richtungen erlaubt.

11. Vorrichtung nach einem der vorstehenden Ansprüche, bei der ein der Röntgenquelle (8) und der Bildaufnahmeeinrichtung (7) zugewandter Bereich der Verkleidung des Ständers (5) auf der Ebene der Liege (1) und darunter so angeschrägt ist, dass die Anschrägung mindestens die Hälfte der der Röntgenquelle (8) und der Bildaufnahmeeinrichtung (7) zugewandten Seite des Ständers (5) umfasst und zu den Verbindungslinien zwischen diesen und dem Ständer (5) einen Winkel zwischen 10° und 60° bildet.

12. Vorrichtung nach einem der vorstehenden Ansprüche, bei der der Ständer (5) ein Geräteregal (9) trägt, dass zumindest teilweise höher als die Patientenliege (1) angeordnet ist.

13. Vorrichtung nach einem der vorstehenden Ansprüche, bei der die Patientenliege (1) eine exzentrische Säule (3) außerhalb der Vertikalprojektion des Abdominalbereichs des Patienten aufweist, an welcher Säule (3) zumindest ein sich entlang der Längsrichtung der Patientenliege (1) mindestens in den Abdominalbereich erstreckender Stützfuß (4) befestigt ist.

14. Vorrichtung nach einem der vorstehenden Ansprüche, bei der der Ständer (5) durch Verfahren mobil ist.

15. Vorrichtung nach einem der vorstehenden Ansprüche, bei der die Patientenliege (1) durch Verfahren mobil ist.

16. Verwendung einer Vorrichtung nach einem der vorstehenden Ansprüche zur Relativanordnung des Ständers (5) und der Patientenliege (1), bei der der Ständer (5) einerseits und die Röntgenquelle (8) und die Bildaufnahmeeinrichtung (7) der Röntgenanlage andererseits bei der vertikalen Durchstrahlung des Abdominalbereichs in vertikaler Projektion eine Verbindungslinie bilden, die zu einer Längsachse der Patientenliege (1) einen Winkel von höchstens 65° bildet.

17. Verwendung nach Anspruch 16, bei der der Ständer (5) außerhalb des Abdominalbereichs, also exzentrisch, neben der Patientenliege (1) angeordnet ist.

18. Verwendung nach Anspruch 17, bei der der Ständer (5) neben dem Fußbereich der Patientenliege (1) angeordnet ist.

19. Verwendung nach Anspruch 16, 17 oder 18 einer Vorrichtung nach Anspruch 5, bei der das Druckwellentherapiegerät (13) entlang dem Bogen (6) so verfahren wird, dass es zwischen einer vertikalen und einer lateralen Behandlungsrichtung verstellt wird.

20. Verwendung nach Anspruch 19, bei der das Druckwellentherapiegerät (13) in einer schrägen Behandlungsposition mit einer Behandlungsrichtung zwischen der vertikalen und der lateralen Behandlungsrichtung verwendet wird.

21. Verwendung nach einem der Ansprüche 16 - 20 einer Vorrichtung nach Anspruch 2, bei der der Bogen (6) durch Verschwenken aus einer vertikalen Durchstrahlungsrichtung in eine schräg-laterale Richtung überführt wird.

22. Verwendung nach einem der Ansprüche 16 - 21 einer Vorrichtung nach Anspruch 4, bei der der Bogen (6) durch Verschwenken und Verdrehen aus einer vertikalen Durchstrahlungsrichtung in eine schräge cranio-caudale Richtung überführt wird.

23. Verwendung nach einem der Ansprüche 16 - 22 einer Vorrichtung nach Anspruch 4, bei der der Bogen (6) durch Verdrehen aus einer vertikalen in eine umgekehrte vertikale Durchstrahlungsposition überführt wird.

24. Verwendung nach einem der Ansprüche 16 - 23, bei der eine zu behandelnde Region in dem Körper durch eine vertikale und eine dazu angeschrägte Röntgenabbildung geortet und die Region danach mit dem Druckwellentherapiegerät (13) beaufschlagt wird.

25. Verwendung nach einem der Ansprüche 16 - 24 einer Vorrichtung nach Anspruch 14, bei der der Ständer (5) mit der Röntgenanlage (7, 8) und dem Druckwellentherapiegerät (13) von einem Behandlungsort zu einem anderen Behandlungsort verfahren wird.

26. Verwendung nach einem der Ansprüche 16 - 25 einer Vorrichtung nach Anspruch 15, bei der die Patientenliege (1) zu einem Behandlungsort neben dem Ständer (5) und nach der Behandlung davon weg verfahren wird.

## Claims

1. An apparatus for shock wave therapy of a human or animal body comprising a focusing shock wave therapy device (13),
an integrated X-ray device (7, 8), an X-ray source (8), and an image capturing device (7) of which being mounted to a stand (5) by carrying arms (6), and
a patient berth (1) beside said stand (5),
said X-ray device (7, 8) and said shock wave therapy device (13) being adapted to be applied to an abdominal region of a patient on said patient berth (1),
said abdominal region being vertically radiographable by said X-ray device (7, 8)
**characterized in that**
said apparatus is adapted to that said stand (5) on the one hand and said X-ray source (8) and said image capturing device (7) of said X-ray device on the other hand, in case of a vertical radiography of said abdominal region and in case of said shock wave therapy device being in a treatment position, have a connection line in a vertical projection in an angle of at most 65° to the longitudinal axis of said patient berth (1),
and that a region of said patient berth (1) corresponding to said abdominal region of said patient is kept free on both sides of that patient berth (1) for accessibility by persons.

2. The apparatus according to claim 1, wherein said X-ray source (8) and said image capturing device (7) of said X-ray device (7, 8) are mounted to arms (6) which are connected to each other in a fixed manner and form an arc (6), which is tiltable on said stand (5) such that in a vertical projection one of said X-ray source (8) and said image capturing device (7) approaches said stand (5) and the other one moves away from said stand (5).

3. The apparatus according to claim 2, wherein said arc (6) is, when tilting together with said X-ray source (8) and said image capturing device (7), tiltable around a fixed horizontal tilting axis, which is perpendicular to connection lines between said X-ray source (8) and said image capturing device (7) on the one hand and said stand (5) on the other hand in case of a vertical radiography and runs through a focus region of said shock wave therapy device (13).

4. The apparatus according to claim 2 or 3, wherein said arc (6) is mounted to said stand (5) rotatably around an axis, which is parallel, in a vertical projection and in case of a vertical radiography of said abdominal region, to connection lines between said X-ray source (8) and said image capturing device (7) on the one hand and said stand (5) on the other hand in case of a vertical radiography and defines an angle to the vertical direction of between 30° and 60°.

5. The apparatus according to one of claims 2 - 4, wherein said shock wave therapy device (13) is mounted to said arc (6) in a manner moveable along said arc (6).

6. The apparatus according to claim 5, wherein said shock wave therapy device (13) is tiltable along said arc (6) around an axis, which is perpendicular to connection lines between said X-ray source (8) and said image capturing device (7) on the one hand and said stand (5) on the other hand in case of a vertical radiography and runs through a focus region of said shock wave therapy device (13).

7. The apparatus according to one of the preceding claims, wherein said shock wave therapy device (13) is moveable into a parking position out of an X-ray path and into a working position by a movement mechanism, wherein in said working position a focus region of said shock wave therapy device (18) is in said X-ray path.

8. The apparatus according to one of the preceding claims, wherein a relative arrangement of said stand (5) and said patient berth (1) is adjustable with regard to two horizontal directions and one vertical direction.

9. The apparatus according of the preceding claims, wherein said shock wave therapy device (13) has a hollow geometry allowing an axial penetration of X-rays.

10. The apparatus according to claim 9, wherein said hollow geometry allows a penetration of X-rays in two mutually angled directions.

11. The apparatus according to one of the preceding claims, wherein a region of the covering of said stand (5) towards said X-ray source (8) and said image capturing device (7) is oblique in the level of said berth (1) and there below such that the oblique form comprises at least half of the side of said stand (5) towards said X-ray source (8) and said image capturing device (7) and defines an angle to connection lines between these and said stand (5) of between 10° and 60°.

12. The apparatus according to one of the preceding claims, wherein an apparatus rack (9) is carried by said stand (5) and is arranged higher than said patient berth (1) at least in part.

13. The apparatus according to one of the preceding claims, wherein said patient berth (1) comprises an eccentric column (3) outside of a vertical projection of said abdominal region of said patient, at which column (3) at least one support foot (4) is mounted being extended along said longitudinal direction of that patient berth (1) at least into said abdominal region.

14. The apparatus according to one of the preceding claims, wherein said stand (5) is mobile by a movement over a floor.

15. The apparatus according to one of the preceding claims, wherein said patient berth (1) is mobile by a movement over a floor.

16. The use of said apparatus according to one of the preceding claims for a relative arrangement of said stand (5) and said patient berth (1), wherein said stand (5) on the one hand and said X-ray source (8) and said image capturing device (7) of said X-ray device on the other hand in case of a vertical radiography of said abdominal region have a connection line in a vertical projection in an angle of at most 65° to a longitudinal axis of said patient berth (1).

17. The use according to claim 16, wherein said stand (5) is arranged outside of said abdominal region, namely eccentrically, beside said patient berth (1).

18. The use according to claim 17, wherein said stand (5) is arranged beside the foot region of said patient berth (1).

19. The use according to claims 16, 17 or 18 of an apparatus according to claim 5, wherein said shock wave therapy device (13) is moved along said arc (6) such that it is adjusted between a vertical and a lateral treatment direction.

20. The use according to claim 19, wherein said shock wave therapy device (13) is used in an oblique treatment position and with a treatment direction between said vertical and said lateral treatment direction.

21. The use according to one of claims 16 - 20 of an apparatus according to claim 2, wherein said arc (6) is transferred from a vertical radiography direction into an oblique-lateral direction by tilting.

22. The use according to one of claims 16 - 21 of an apparatus according to claim 4, wherein said arc (6) is transferred from a vertical radiography direction into an oblique cranio-caudal direction by tilting and rotating.

23. The use according to one of claims 16 - 22 of an apparatus according to claim 4, wherein said arc (6) is transferred from a vertical into an inverted vertical radiography position by rotating.

24. The use according to one of claims 16 - 23, wherein a region to be treated in said body is located by a vertical X-ray imaging and a relatively thereto oblique X-ray imaging and wherein said region is treated by said shock wave therapy device (13) thereafter.

25. The use according to one of claims 16 - 24 of an apparatus according to claim 14, wherein said stand (5) is moved together with said X-ray device (7, 8) and said shock wave therapy device (13) from one treatment location to another treatment location.

26. The use according to one of claims 16 - 25 of an apparatus according to claim 15, wherein said patient berth (1) is moved to a treatment location between said stand (5) and away therefrom after said treatment.

## Revendications

1. Dispositif pour la thérapie par ondes de choc sur un corps d'humain ou d'animal, avec
un appareil thérapeutique à ondes de choc (13),
un système radiographique intégré (7, 8), dont la source radiographique (8) et le dispositif d'enregistrement d'images (7) sont respectivement fixés à des bras de support (6) sur un portant (5),
et
un lit de patient (1) situé à côté du portant (5),
dans lequel le dispositif radiographique (7, 8) et l'appareil thérapeutique à ondes de choc (13) sont conçus pour la détection d'une zone abdominale d'un patient couché sur le lit (1),
la zone abdominale peut être traversée verticalement par le rayonnement à l'aide du dispositif radiographique (7, 8),
**caractérisé en ce que**
le dispositif est conçu de telle manière que le portant (5) d'une part et la source radiographique (8) et le dispositif d'enregistrement d'images (7) du système radiographique d'autre part forment une ligne de liaison lors du rayonnement vertical à travers la zone abdominale et lorsque l'appareil thérapeutique à ondes de choc (13) se trouve dans une position de traitement, formant un angle de 65° maximum par rapport à l'axe longitudinal du lit de patient (1), et
**en ce que** le dispositif laisse une zone du lit de patient (1), correspondant à la zone abdominale du patient, dégagée des deux côtés du lit de patient (1), pour permettre à une personne d'y accéder.

2. Dispositif selon la revendication 1, dans lequel la source radiographique (8) et le dispositif d'enregistrement d'images (7) du système radiographique sont fixés à des bras (6) reliés fixement entre eux, qui forment un arc (6) susceptible d'être pivoté sur le portant (5), si bien que lors d'une projection verticale, l'un parmi la source radiographique (8) et le dispositif d'enregistrement d'images (7) se rapproche du portant (5) et l'autre s'éloigne du portant (5).

3. Dispositif selon la revendication 2, dans lequel l'arc (6) est pivotable autour d'un axe de pivotement horizontal fixe lors d'un pivotement avec la source radiographique (8) et le dispositif d'enregistrement d'images (7), ledit axe de pivotement étant perpendiculaire aux lignes de liaison entre la source radiographique (8) et le dispositif d'enregistrement d'images (7) d'une part et le portant (5) d'autre part, lors d'un rayonnement vertical en projection verticale, et croisant une zone de focalisation de l'appareil thérapeutique à ondes de choc (13).

4. Dispositif selon l'une des revendications 2 ou 3, dans lequel l'arc (6) est monté sur le portant (5) de manière à pouvoir tourner autour d'un axe (14) qui est parallèle aux lignes de liaison entre la source radiographique (8) et le dispositif d'enregistrement d'images (7) d'une part et le portant (5) d'autre part lors du rayonnement vertical en projection verticale, et qui forme un angle compris entre 30° et 60° par rapport à la verticale.

5. Dispositif selon l'une des revendications 2 à 4, dans lequel l'appareil thérapeutique à ondes de choc (13) est monté sur l'arc (6) et peut être déplacé le long de l'arc (6).

6. Dispositif selon la revendication 5, dans lequel l'appareil thérapeutique à ondes de choc (13) est pivotable le long de l'arc (6), autour d'un axe horizontal, qui est perpendiculaire aux lignes de liaison entre la source radiographique (8) et le dispositif d'enregistrement d'images (7) d'une part et le portant (5) d'autre part lors d'un rayonnement vertical, et qui croise une zone de focalisation de l'appareil thérapeutique à ondes de choc (13).

7. Dispositif selon l'une des revendications précédentes, dans lequel l'appareil thérapeutique à ondes de choc (13) est déplacable dans une position de stationnement en-dehors du parcours des rayons X et dans une position de travail, à l'aide d'un mécanisme de déplacement, la zone de focalisation de l'appareil thérapeutique à ondes de choc (13) se trouvant sur le parcours des rayons X dans la position de travail.

8. Dispositif selon l'une des revendications précédentes, dans lequel la disposition relative du support (5) et du lit de patient (1) peut être réglée dans deux directions horizontales et dans une direction verticale.

9. Dispositif selon l'une des revendications précédentes, dans lequel l'appareil thérapeutique à ondes de choc (13) présente une géométrie creuse permettant un passage axial des rayons X.

10. Dispositif selon la revendication 9, dans lequel la géométrie creuse permet le passage des rayons X dans deux directions coudées l'une par rapport à l'autre.

11. Dispositif selon l'une des revendications précédentes, dans lequel une zone de l'habillage du portant (5) qui est tournée vers la source radiographique (8) et le dispositif d'enregistrement d'images (7) est chanfreinée de telle façon sur le plan du lit (1) et en-dessous, que le chanfrein comprend au moins la moitié du côté du portant (5) qui est tourné vers la source radiographique (8) et le dispositif d'enregistrement d'images (7), et forme un angle compris entre 10° et 60° par rapport aux lignes de liaison entre ceux-ci et le portant (5).

12. Dispositif selon l'une des revendications précédentes, dans lequel le portant (5) porte une étagère d'appareil (9), qui est disposée au moins partiellement plus en hauteur que le lit de patient (1).

13. Dispositif selon l'une des revendications précédentes, dans lequel le lit de patient (1) comporte une colonne excentrée (3) en-dehors de la projection verticale de la zone abdominale du patient, sur laquelle colonne (3) est fixé au moins un pied d'appui (4) qui s'étend au moins dans la zone abdominale, le long du sens longitudinal du lit de patient (1).

14. Dispositif selon l'une des revendications précédentes, dans lequel le portant (5) est mobile par déplacement.

15. Dispositif selon l'une des revendications précédentes, dans lequel le lit de patient (1) est mobile par déplacement.

16. Utilisation d'un dispositif selon l'une des revendications précédentes, pour la disposition relative du portant (5) et du lit de patient (1), dans lequel le portant (5) d'une part et la source radiographique (8) et le dispositif d'enregistrement d'images (7) du système radiographique d'autre part forment, lors d'un rayonnement vertical à travers la zone abdominale en projection verticale, une ligne de liaison formant un angle de 65° maximum par rapport à un axe longitudinal du lit de patient (1).

17. Utilisation selon la revendication 16, dans laquelle le portant (5) est disposé en-dehors de la zone abdominale, c'est-à-dire de façon excentrée, à côté du lit de patient (1).

18. Utilisation selon la revendication 17, dans laquelle le portant (5) est disposé à côté de la zone de pied du lit de patient (1).

19. Utilisation selon l'une des revendications 16, 17 ou 18, d'un dispositif selon la revendication 5, dans laquelle l'appareil thérapeutique à ondes de choc (13) est déplacé de telle façon le long de l'arc (6), qu'il est réglé entre une direction de traitement verticale et une direction de traitement latérale.

20. Utilisation selon la revendication 19, dans laquelle l'appareil thérapeutique à ondes de choc (13) est utilisé dans une position de traitement inclinée, avec une direction de traitement entre la direction de traitement verticale et la direction de traitement latérale.

21. Utilisation selon l'une des revendications 16 à 20 d'un dispositif selon la revendication 2, dans laquelle l'arc (6) est basculé dans une direction oblique-latérale par pivotement hors d'une direction de rayonnement verticale.

22. Utilisation selon l'une des revendications 16 à 21 d'un dispositif selon la revendication 4, dans laquelle l'arc (6) est basculé dans une direction crânio-caudale oblique, par pivotement et rotation hors d'une direction de rayonnement verticale.

23. Utilisation selon l'une des revendications 16 à 22 d'un dispositif selon la revendication 4, dans laquelle l'arc (6) est basculé dans une position de rayonnement verticale inversée, par rotation hors d'une direction verticale.

24. Utilisation selon l'une des revendications 16 à 23, dans laquelle une région du corps à traiter est localisée par une image radiographique verticale et inclinée, et la région est ensuite soumise à l'appareil thérapeutique à ondes de choc (13).

25. Utilisation selon l'une des revendications 16 à 24 d'un dispositif selon la revendication 14, dans laquelle le portant (5) est déplacé d'un site de traitement à un autre site de traitement avec le système radiographique (7, 8) et l'appareil thérapeutique à ondes de choc (13).

26. Utilisation selon l'une des revendications 16 à 25 d'un dispositif selon la revendication 15, dans laquelle le lit de patient (1) est déplacé vers un site de traitement à côté du portant (5), puis éloigné à la fin du traitement.
